# EUROPEAN PATENT SPECIFICATION

(11) **EP 2 997 956 B1**
(45) Date of publication and mention of the grant of the patent: **26.04.2017**
(21) Application number: 14797849.8
(22) Date of filing: 07.05.2014
(51) Int. Cl.: A61K 8/27, A61K 8/06, A61K 8/29, A61K 8/31, A61K 8/35, A61K 8/49, A61K 8/58, A61K 8/81, A61K 8/89, A61K 8/891, A61K 8/894, A61Q 17/04

(54) **WATER-IN-OIL COSMETIC**
WASSER-IN-ÖL-KOSMETIKUM
PRODUIT COSMÉTIQUE TYPE L'EAU DANS L'HUILE

(30) Priority: 15.05.2013 JP 2013103487
(43) Date of publication of application: 23.03.2016
(73) Proprietor: FUJIFILM Corporation, Tokyo 106-8620 (JP)
(72) Inventor: AOKI, Minako, Ashigarakami-gun Kanagawa 258-8577 (JP); SAKAGUCHI, Hiroyuki, Ashigarakami-gun Kanagawa 258-8577 (JP)
(74) Representative: Klunker . Schmitt-Nilson . Hirsch
(86) International application number: PCT/JP2014/062281
(87) International publication number: WO 2014/185316

(56) References cited:
- WO-A1-2010/098249
- WO-A1-2013/002278
- WO-A1-2013/065643
- WO-A1-2013/065644
- JP-A- 2012 211 114

## Description

### BACKGROUND OF THE INVENTION

### 1. Field of the Invention

The present invention relates to a water-in-oil cosmetic.

### 2. Description of the Related Art

Thus far, a variety of organic ultraviolet absorbents have been used to impart an ultraviolet ray shielding effect to cosmetics, and particularly, ultraviolet ray shielding powder obtained by complexing an ultraviolet absorbent which is an organic compound with inorganic powder has been known.

For example, WO2010/098249A discloses complex powder obtained by coating the surface of powder serving as a base material with an organic compound having an ultraviolet ray absorbing function. In WO2010/098249A, it is described that this complex powder is obtained by complexing an organic compound having an ultraviolet ray absorbing function and an inorganic colorant having ultraviolet ray scattering function, is powder that can be stably formulated into cosmetics, and is an organic ultraviolet absorbent capable of maintaining a strong ultraviolet ray absorbing effect and having favorable dispersibility. WO2010/098249A discloses complex powder obtained by coating the surface of a titanium oxide fine particle with butyl methoxy dibenzoyl methane as the above-described complex powder.

Meanwhile, as a natural component capable of exhibiting a variety of functions, a number of cosmetics containing a carotenoid are known. Since a carotenoid has properties of being easily decomposed by light or the like, it is required to stably add a carotenoid to a cosmetic.

For example, JP2012-211114A discloses a water-in-oil emulsified cosmetic into which hydrophobilized inorganic powder such as dimethyl polysiloxane-treated titanium oxide, a specific silicone-based surfactant, silicone oil, triglyceride, and astaxanthin are formulated. It is described that this water-in-oil emulsified cosmetic is well spread even when formulated with powder and has excellent temporal stability of astaxanthin even when coexisting with a powder component.

### SUMMARY OF THE INVENTION

However, when powder having butyl methoxy benzoyl methane on the surface is formulated into an emulsified substance, there are cases in which feelings during use such as the unevenness of the skin becoming highly visible are impaired. In addition, there is still room for improving the temporal stability of carotenoids in the presence of powder having butyl methoxy benzoyl methane on the surface. As a result, there is an increasing demand for having an excellent feeling during use and satisfying both a favorable ultraviolet ray shielding function and a favorable temporal stability of carotenoids.

An object of the present invention is to provide a water-in-oil cosmetic having a favorable ultraviolet ray shielding effect, an excellent temporal stability of carotenoids, and a favorable feeling during use.

The present invention is as described below.
[1] A water-in-oil cosmetic including: surface-treated powder including at least one oxide selected from a group consisting of titanium oxide and zinc oxide and having 4-tert-butyl-4-methoxy benzoyl methane on a surface; at least one carotenoid; at least one sorbitan fatty acid ester; at least one polyether-modified silicone; and at least one compound selected from a group consisting of a coating-forming silicone polymer and an alkyl (meth)acrylate polymer.
[2] The water-in-oil cosmetic according to [1], in which the polyether-modified silicone is a polyether-modified silicone having a straight chain or a branched chain.
[3] The water-in-oil cosmetic according to [1] or [2], in which the polyether-modified silicone is a polyether-modified silicone having a branched chain.
[4] The water-in-oil cosmetic according to any one of [1] to [3], in which the sorbitan fatty acid ester is at least one sorbitan fatty acid ester selected from a group consisting of sorbitan oleate, sorbitan isostearate, sorbitan sesquioleate, and sorbitan sesquiisostearate.
[5] The water-in-oil cosmetic according to any one of [1] to [4], in which the polyether-modified silicone is at least one polyether-modified silicone selected from a group consisting of PET-9 polydimethylsiloxyethyl dimethicone and lauryl PET-9 polydimethylsiloxyethyl dimethicone.
[6] The water-in-oil cosmetic according to any one of [1] to [5], in which the compound selected from the group consisting of a coating-forming silicone polymer and an alkyl (meth)acrylate polymer is at least one compound selected from a group consisting of trimethylsiloxysilicate, polymethylsilsesquioxane, an alkyl acrylate/dimethicone copolymer, and an alkyl acrylate copolymer.
[7] The water-in-oil cosmetic according to any one of [1] to [6], in which a total content of the compound selected from the group consisting of a coating-forming silicone polymer and an alkyl (meth)acrylate polymer is in a range of 0.1% by mass to 15% by mass.
[8] The water-in-oil cosmetic according to any one of [1] to [7], in which the carotenoid is at least one carotenoid selected from lycopene, astaxanthin, and derivatives thereof.
[9] The water-in-oil cosmetic according to any one of [1] to [8], in which the carotenoid is astaxanthin.

According to the present invention, it is possible to provide a water-in-oil cosmetic having a favorable ultraviolet ray shielding effect, an excellent temporal stability of carotenoids, and a favorable feeling during use.

### DESCRIPTION OF THE PREFERRED EMBODIMENTS

A water-in-oil cosmetic of the present invention includes (a) surface-treated powder including at least one oxide selected from a group consisting of titanium oxide and zinc oxide and having 4-tert-butyl-4-methoxy benzoyl methane on a surface, (b) at least one carotenoid, (c) at least one sorbitan fatty acid ester, (d) at least one polyether-modified silicone, and (e) at least one compound selected from a group consisting of a coating-forming silicone polymer and an alkyl (meth)acrylate polymer.

Since the water-in-oil cosmetic of the present invention includes a combination of predetermined surface-treated powder having 4-tert-butyl-4-methoxy benzoyl methane on the surface, at least one carotenoid, at least one sorbitan fatty acid ester, at least one polyether-modified silicone, and at least one compound selected from a group consisting of a coating-forming silicone polymer and an alkyl (meth)acrylate polymer, a favorable ultraviolet ray shielding effect, an excellent temporal stability of carotenoids, and a favorable feeling during use can be obtained.

That is, it has been found that the specific surface-treated powder having 4-tert-butyl-4-methoxy benzoyl methane on the surface is surface-treated powder having a favorable ultraviolet ray shielding function and contributes to improvement in the temporal stability of carotenoids. In addition, when a sorbitan fatty acid ester, a polyether-modified silicone, and a compound selected from a group consisting of a coating-forming silicone polymer and an alkyl (meth)acrylate polymer are further formulated into the water-in-oil cosmetic, it is assumed that the dispersibility of the specific surface-treated powder improves, accordingly, the feeling of the water-in-oil cosmetic during use improves, and additionally, the temporal stability of carotenoids further improves. However, the present invention is not confined by any specific theories.

Generally, water-in-oil cosmetics including the surface-treated powder are often felt heavy when applied to the skin due to the surface-treated powder. In water-in-oil cosmetics including the surface-treated powder, there are cases in which the surface-treated powder is unevenly spread depending on the coating direction on a surface being coated or the movement of the hand or the like, the surface-treated powder is localized on the surface coated with the water-in-oil cosmetic, and the ultraviolet ray shielding effect is degraded. In contrast, in the present invention, it is considered that, since the surface-treated powder is combined with a sorbitan fatty acid ester and a polyether-modified silicone, the dispersibility of the surface-treated powder significantly improves, consequently, the water-in-oil cosmetic is not felt heavy when applied to the skin, and a water-in-oil cosmetic having a favorable feeling during use can be provided. In the present invention, since at least one of a coating-forming silicone polymer and an alkyl (meth)acrylate polymer is included in addition to the above-described components, it is assumed that the dispersibility of the surface-treated powder becomes more favorable, the surface-treated powder is evenly spread on an application subject, for example, the skin, and an effect of preventing the ultraviolet ray shielding effect from being degraded even after a certain period of time elapses can be obtained. However, the present invention is not confined by any specific theories.

In the present specification, the term "step" refers not only to an independent step but also to a step which cannot be clearly distinguished from other steps as long as a desired effect of the step can be achieved.

In the present invention, a numerical range expressed using "to" refers to a range including the numerical value before and after the "to" as the upper limit value and the lower limit value respectively.

In the present specification, the amount of individual components in a composition refers to, in a case in which the composition includes a plurality of substances which correspond to the individual components, the total amount of the plurality of substances in the composition unless particularly otherwise described.

In the present specification, an "oil-phase component" refers to a component which can be used to prepare an oil-phase composition by being combined with an oil solution when preparing the water-in-oil cosmetic and can be present in a state of being dispersed or dissolved in a continuous phase of the water-in-oil cosmetic (a water droplet is not included).

In the present specification, a "water-phase component" refers to a component which can be used to prepare a water-phase composition by being combined with an aqueous medium such as water when preparing the water-in-oil cosmetic and can be present in a state of being dispersed or dissolved in a water droplet of the water-in-oil cosmetic.

In the present specification, "(meth)acrylic acid" refers to both or either acrylic acid and methacrylic acid.

### Hereinafter, the present invention will be described.

The water-in-oil cosmetic of the present invention includes surface-treated powder including at least one oxide selected from a group consisting of titanium oxide and zinc oxide and having 4-tert-butyl-4-methoxy benzoyl methane on the surface, at least one carotenoid, at least one sorbitan fatty acid ester, at least one polyether-modified silicone, and at least one compound selected from a group consisting of a coating-forming silicone polymer and an alkyl (meth)acrylate polymer and includes other components as necessary.

### (a) Surface-treated powder

The surface-treated powder in the present invention is surface-treated powder including at least one oxide selected from a group consisting of titanium oxide (TiO₂) and zinc oxide (ZnO) and having 4-tert-butyl-4-methoxy benzoyl methane on the surface (hereinafter, in some cases, simply referred to as "surface-treated powder"). Since the surface-treated powder includes 4-tert-butyl-4-methoxy benzoyl methane, the surface-treated powder has a favorable ultraviolet ray shielding function.

The surface-treated powder includes at least one oxide selected from a group consisting of titanium oxide and zinc oxide and, from the viewpoint of an ultraviolet ray protecting effect obtained from a difference in the ultraviolet ray shielding wavelength range from 4-tert-butyl-4-methoxy benzoyl methane, the surface-treated powder is preferably titanium oxide. In the present specification, in some cases, at least one oxide selected from a group consisting of titanium oxide and zinc oxide will be collectively referred to as "inorganic powder".

The crystal form of the titanium oxide may be any of anatase, rutile, and brookite. The crystal form of the titanium oxide is preferably rutile. In a case in which the crystal form of the titanium oxide is a rutile form, it is possible to more effectively shield ultraviolet rays, which is preferable.

The zinc oxide preferably has a wurtzite-form crystal structure.

Any inorganic powder which can be generally used for cosmetic products can be used without any particular limitations depending on the shape such as a spherical shape, a plate shape, or a needle shape, the particle diameter such as a haze form, fine particles, or a colorant class, the particle structure such as a porous structure or a pore-free structure, and the like.

From the viewpoint of the ultraviolet ray shielding function, the average primary particle diameter of the inorganic powder is preferably 200 nm or smaller. From the viewpoint of the transparency and the feeling during use of the cosmetic, the average primary particle diameter of the inorganic powder is preferably in a range of 1 nm to 90 nm and more preferably in a range of 5 nm to 50 nm. As the average primary particle diameter of the inorganic powder, the average value obtained by dispersing the inorganic powder, then, capturing an image of 1000 or more powder particles using a transmission electron microscope, carrying out an imaging process on individual particles in the captured image using an image analysis-type particle size distribution analyzer, and measuring the circle-equivalent diameters of the particles is used. In a case in which a commercially available product is used, the average primary particle diameter of the inorganic powder used in the commercially available product can be applied with no changes as the average primary particle diameter of the inorganic powder.

As the inorganic powder, it is possible to use, for example, inorganic powder having a surface treated with an inorganic substance such as aluminum oxide, silicon oxide, or zirconium oxide, inorganic powder having a surface treated with an organic substance such as stearic acid or the like in order to suppress the surface activity of the inorganic powder. As the inorganic powder, it is possible to use inorganic powder obtained by further carrying out a hydrophobilization treatment such as a silicone treatment, a fatty acid treatment, a metal soap treatment, or a fluorine treatment or a hydrophilization treatment such as a polyacrylic acid treatment on the inorganic powder having a surface treated with an inorganic substance or the inorganic powder having a surface treated with an organic substance.

The surface-treated powder has 4-tert-butyl-4-methoxy benzoyl methane on the surface. The surface-treated powder preferably has 4-tert-butyl-4-methoxy benzoyl methane on the entire outermost surface. However, on the outermost surface of the surface-treated powder, there may be a portion having 4-tert-butyl-4-methoxy benzoyl methane as long as the effect of the present invention is not impaired. Regarding the bonding pattern between the surface of the surface-treated powder (in a case in which the surface of the inorganic powder is further treated with an inorganic substance or an organic substance, the inorganic substance or the organic substance disposed on the surface) and 4-tert-butyl-4-methoxy benzoyl methane, there is no particular limitation as long as 4-tert-butyl-4-methoxy benzoyl methane and the inorganic powder integrally behave. The bonding pattern may be a chemical bond such as a covalent bond or a non-chemical bond such as adsorption.

The presence of 4-tert-butyl-4-methoxy benzoyl methane on the surface of the surface-treated powder can be confirmed by adding ethanol to the surface-treated powder, stirring ethanol and the surface-treated powder together, and measuring the absorbance of a supernatant solution at a wavelength of 355 nm using a spectrophotometer.

The average particle diameter of the surface-treated powder is preferably smaller than 1 µm. When the average particle diameter of the surface-treated powder is smaller than 1 µm, it is considered that the cosmetic being colored by the surface-treated powder is suppressed and a so-called white-turbidity phenomenon tends not to occur. From the viewpoint of the ultraviolet ray shielding function and the feeling during use, the average particle diameter of the surface-treated powder is more preferably in a range of 1 nm to 500 nm and still more preferably in a range of 3 nm to 100 nm.

The average particle diameter of the surface-treated powder can be measured using the same method as for the average primary particle diameter.

Examples of the surface-treated powder include the surface-treated powder disclosed in WO2010/098249A. As the surface-treated powder, it is possible to use HXMT-100ZA (manufactured by TAYCA Corporation), HXMT-10EXA (manufactured by TAYCA Corporation), HXLT-02 (manufactured by TAYCA Corporation), or the like which can be procured from commercially available products.

In the water-in-oil cosmetic of the present invention, the content of the surface-treated powder is not particularly limited, but is preferably in a range of 0.1% by mass to 30% by mass. When the content of the surface-treated powder being used is in the above-described range, it is possible to obtain a water-in-oil cosmetic in which the feeling during use such as a feeling of the spreading of the cosmetic when applying the cosmetic, the temporal stability of the water-in-oil cosmetic, and the temporal stability of carotenoids are more favorable.

From the viewpoint of the feeling during use and the temporal stability of carotenoids, the content of the surface-treated powder is preferably in a range of 0.1% by mass to 50% by mass, more preferably in a range of 1% by mass to 30% by mass, still more preferably in a range of 2% by mass to 20% by mass, and most preferably in a range of 3% by mass to 10% by mass.

From the viewpoint of the temporal stability of carotenoids, the content of the surface-treated powder to the content of the carotenoid in terms of mass is preferably in a range of 1 time to 400000 times, more preferably in a range of 10 times to 40000 times, and still more preferably in a range of 100 times to 4000 times. When the content of the surface-treated powder to the content of the carotenoid in terms of mass is 400000 times or lower, there is a tendency that the temporal stability of carotenoids is further improved.

The surface-treated powder is included in an oil phase or a water phase in the water-in-oil cosmetic. From the viewpoint of the stability of the cosmetic, the surface-treated powder is preferably included in an oil phase. When the surface-treated powder is included in an oil phase, there is a tendency that the surface-treated powder can be more stably formulated with the oil phase in a case in which a larger amount of the surface-treated powder is used, particularly, in a case in which the content of the surface-treated powder reaches 20% by mass or higher of an water-in-oil cosmetic.

In a case in which the water-in-oil cosmetic includes the surface-treated powder in an oil phase, for example, it is also possible to prepare a slurry including the surface-treated powder using a volatile oil component such as a silicone oil as a dispersion medium and formulate the surface-treated powder with other components in a slurry form.

The content of the surface-treated powder in the slurry is not particularly limited; however, generally, the content thereof is preferably set in a range of 10% by mass to 80% by mass and more preferably set in a range of 20% by mass to 60% by mass of the total mass of the slurry. When the content of the surface-treated powder in the slurry is 10% by mass or higher of the total mass of the slurry, it is possible to more stably formulate the surface-treated powder with the water-in-oil cosmetic, which is preferable. When the content of the surface-treated powder in the slurry is in a range of 20% by mass to 60% by mass of the total mass of the slurry, it is possible to more stably formulate the surface-treated powder with the water-in-oil cosmetic, which is preferable.

### (b) Carotenoid

The water-in-oil cosmetic of the present invention includes at least one carotenoid. Carotenoids refer to pigments of yellow to red terpenoids, and examples thereof include carotenoids derived from plants, algae, and bacteria. The carotenoids are not limited to naturally-occurring carotenoids and may be any carotenoids obtained according to an ordinary method.

Specific examples of the carotenoid in the present invention include lycopene, α-carotene, β-carotene, γ-carotene, δ-carotene, actinioerythrol, bixin, canthaxanthin, capsorubin, β-8'-apo-carotenal (apocarotenal), β-12'-apo-carotenal, xanthophylls (for example, astaxanthin, fucoxanthin, lutein, zeaxanthin, capsanthin, β-cryptoxanthin, violaxanthin, violerythrin, and the like), and hydroxyl derivatives or carboxyl derivatives thereof. Only one carotenoid may be used or a combination of two or more carotenoids may be used. Among these, astaxanthin and lycopene are preferred, and particularly, astaxanthin having an anti-aging effect, an anti-inflammatory effect, and a whitening effect is preferred.

From the viewpoint of efficient development of the functions of the carotenoid, favorable compatibility with the skin, and efficient obtainment of a white-turbidity-preventing effect, the total content of the carotenoid in the water-in-oil cosmetic is preferably in a range of 0.000001% by mass to 1% by mass, more preferably in a range of 0.00001% by mass to 0.1% by mass, and still more preferably in a range of 0.0001% by mass to 0.05% by mass.

### (c) Sorbitan fatty acid ester

The water-in-oil cosmetic of the present invention includes at least one sorbitan fatty acid ester. When the water-in-oil cosmetic of the present invention includes a sorbitan fatty acid ester, the sorbitan fatty acid ester acts as an oil-soluble emulsifier component, and the temporal stability of carotenoids improves.

The sorbitan fatty acid ester is preferably a sorbitan fatty acid ester in which the fatty acid has 8 or more carbon atoms and more preferably a sorbitan fatty acid ester in which the fatty acid has 12 or more carbon atoms.

Examples of the sorbitan fatty acid ester include sorbitan monolaurate, sorbitan monopalmitate, sorbitan monostearate, sorbitan monooleate, sorbitan monoisostearate, sorbitan sesquicaprylate, sorbitan sesquioleate, sorbitan sesquistearate, sorbitan sesquiisostearate, sorbitan trioleate, sorbitan tristearate, sorbitan triisostearate, diglycerol sorbitan tetra-2-ethylhexanoate, diglycerol sorbitan penta-2-ethylhexanoate, and sorbitan cocoate. From the viewpoint of the temporal stability of carotenoids and the feeling during use, the sorbitan fatty acid ester is particularly preferably sorbitan monooleate, sorbitan monoisostearate, sorbitan sesquioleate, sorbitan sesquiisostearate, or a combination thereof. In the present invention, it is possible to use only one sorbitan fatty acid ester or a combination of two or more sorbitan fatty acid esters.

From the viewpoint of the dispersibility of the surface-treated powder and the feeling during use, the total content of the sorbitan fatty acid ester is preferably set in a range of 0.01% by mass to 20% by mass, more preferably set in a range of 0.01 % by mass to 15% by mass, still more preferably in a range of 0.05% by mass to 10% by mass, and even still more preferably in a range of 0.1% by mass to 5% by mass of the total mass of the cosmetic. When the total content of the sorbitan fatty acid ester is 0.01% by mass or higher of the total mass of the cosmetic, the dispersibility of the surface-treated powder is enhanced, and there is a tendency that the dispersibility of the water-in-oil cosmetic is further improved, and, when the total content of the sorbitan fatty acid ester is 20% by mass or lower, there is a tendency that the feeling of the water-in-oil cosmetic during use can be more favorably maintained.

In addition, the total content of the sorbitan fatty acid ester to the content of the surface-treated powder in terms of mass is preferably in a range of 0.0001 times to 150 times, more preferably in a range of 0.0005 times to 50 times, still more preferably in a range of 0.001 times to 5 times, even still more preferably in a range of 0.001 times to 1 time, and particularly preferably in a range of 0.001 times to 0.5 times. When the total content of the sorbitan fatty acid ester to the total content of the surface-treated powder in terms of mass is 0.0001 times or higher, the dispersibility of the surface-treated powder is enhanced, and there is a tendency that the temporal stability of the carotenoid is further improved, and, when the total content of the sorbitan fatty acid ester is 150 times or lower, there is a tendency that the dispersibility of the surface-treated powder is further improved.

### (d) Polyether-modified silicone

The water-in-oil cosmetic of the present invention includes at least one polyether-modified silicone. When the water-in-oil cosmetic of the present invention includes a polyether-modified silicone, the polyether-modified silicone is included in an oil phase of the water-in-oil emulsification composition, and the temporal stability of carotenoids improves.

The polyether-modified silicone is a polymer having a silicone chain and a polyether chain introduced through modification. Examples of the polyether chain include polyethylenen glycol and polypropylene glycol.

The silicone chain may be a linear silicone chain or a silicone chain having a branched chain. Examples of the branched chain that the silicone chain can have include an alkyl chain having 8 to 22 carbon atoms, an alkenyl chain having 8 to 22 carbon atoms, and a polyethylenen glycol (PEG) chain having a degree of polymerization in a range of 1 to 50. The silicone chain may have one or two or more branched chains.

From the viewpoint of the temporal stability of the cosmetic, the polyether-modified silicone is preferably a polyether-modified silicone having a branched chain, and more preferably, the branched chain is a PEG chain.

The HLB of the polyether-modified silicone is preferably in a range of 1 to 10 and more preferably in a range of 2 to 8. When the HLB of the polyether-modified silicone is 10 or lower, the emulsification stability of the water-in-oil cosmetic further improves.

Examples of the polyether-modified silicone having an HLB in a range of 1 to 10 include PEG-11 methyl ether dimethicone, PEG/PPG-20/22 butyl ether dimethicone, PEG/PPG-19/19 dimethicone, PEG-9 dimethicone, PEG-9 methyl ether dimethicone, PEG-10 dimethicone, PEG-21 methyl ether dimethicone, PEG-9 polydimethylsiloxyethyl dimethicone, and lauryl PEG-9 polydimethylsiloxyethyl dimethicone.

In the water-in-oil cosmetic, it is possible to use only one polyether-modified silicone or a combination of two or more polyether-modified silicones.

The polyether-modified silicone can be procured from commercially available products, and examples thereof include SILICONE KF-6011, KF-6011P, KF-6012, KF-6013, KF-6015, KF-6016, KF-6017, KF-6017P, KF-6043, PK-6004, KF-6028, KF-6028P, and KF-06038 all manufactured by Shin-Etsu Chemical Co., Ltd.

From the viewpoint of the temporal stability of the cosmetic, the polyether-modified silicone is preferably a polyether-modified silicone having a branched chain, and examples thereof include PEG-9 polydimethylsiloxyethyl dimethicone and lauryl PEG-9 polydimethylsiloxyethyl dimethicone. Among these, the polyether-modified silicone is more preferably at least one polyether-modified silicone selected from a group consisting of PEG-9 polydimethylsiloxyethyl dimethicone and lauryl PEG-9 polydimethylsiloxyethyl dimethicone.

From the viewpoint of the temporal stability of the cosmetic, the total content of the polyether-modified silicone in the water-in-oil cosmetic is preferably set in a range of 0.01% by mass to 10% by mass, more preferably set in a range of 0.1 % by mass to 8% by mass, and still more preferably in a range of 0.5% by mass to 6% by mass of the total mass of the cosmetic. When the total content of the polyether-modified silicone is 0.01% by mass or higher of the total mass of the cosmetic, the dispersibility of the surface-treated powder improves, and there is a tendency that the temporal stability of carotenoids further improves.

### (e) At least one compound selected from group consisting of coating-forming silicone polymer and alkyl (meth)acrylate polymer

The water-in-oil cosmetic of the present invention includes at least one compound selected from a group consisting of a coating-forming silicone polymer and an alkyl (meth)acrylate polymer (hereinafter, in some cases, simply collectively referred to as "e component"). When the water-in-oil cosmetic includes at least one compound selected from a group consisting of a coating-forming silicone polymer and an alkyl (meth)acrylate polymer, it is possible to prevent the aggregation of the surface-treated powder and to enhance the temporal stability of the water-in-oil cosmetic.

As the coating-forming silicone polymer, a silicone resin can be used. Examples of the coating-forming silicone polymer include trimethylsiloxysilicate, polymethylsilsesquioxane, polypropylsilsesquioxane, an acrylates/polytrimethylsiloxymethacrylate copolymer, and an alkyl acrylate/dimethicone copolymer. In the water-in-oil cosmetic, it is possible to use only one coating-forming silicone polymer or a combination of two or more coating-forming silicone polymers. Among these, from the viewpoint of the dispersibility of the surface-treated powder, trimethylsiloxysilicate, polymethylsilsesquioxane, or an alkyl acrylate/dimethicone copolymer is preferred.

Examples of the alkyl (meth)acrylate polymer include an alkyl acrylate copolymer, a methyl methacrylate crosspolymer, a methyl methacrylate/glycol dimethacrylate crosspolymer, a butyl acrylate/glycol dimethacrylate crosspolymer, an ammonium acrylates copolymer, and an acrylates/ethylhexyl acrylate copolymer. Examples of an alkyl group that can be included in the alkyl (meth)acrylate copolymer as a copolymerization component include an alkyl group having 1 to 3 carbon atoms, and, from the viewpoint of the dispersibility of the surface-treated powder, an alkyl group having 1 to 10 carbon atoms is more preferred. In the water-in-oil cosmetic, it is possible to use only one alkyl (meth)acrylate polymer or a combination of two or more alkyl (meth)acrylate polymers. Among these, from the viewpoint of the dispersibility of the surface-treated powder and the temporal stability of the cosmetic, an alkyl acrylate copolymer is preferred, and an alkyl acrylate copolymer having an alkyl group with 1 to 10 carbon atoms is more preferred.

From the viewpoint of the dispersibility of the surface-treated powder and ease of forming a coating after the application of the cosmetic, the compound selected from a group consisting of the coating-forming silicone polymer and the alkyl (meth)acrylate polymer is preferably at least one compound selected from a group consisting of trimethylsiloxysilicate, polymethylsilsesquioxane, an alkyl acrylate/dimethicone copolymer, and an alkyl acrylate copolymer.

From the viewpoint of the temporal stability, the total content of the compound selected from the group consisting of the coating-forming silicone polymer and the alkyl (meth)acrylate polymer in the water-in-oil cosmetic is preferably set in a range of 0.01% by mass to 30% by mass, more preferably set in a range of 0.05% by mass to 20% by mass, and still more preferably in a range of 0.1% by mass to 15% by mass of the total mass of the cosmetic. When the total content of the e component is 0.05% by mass or higher of the total mass of the cosmetic, there is a tendency that the formation of a coating after the application of the cosmetic becomes easy, and, when the total content thereof is 15% by mass or lower, there is a tendency that the dispersion stability of the surface-treated powder can be obtained.

### (f) Water

The water-in-oil cosmetic of the present invention includes water.

The water that the water-in-oil cosmetic of the present invention includes is not particularly limited as long as the water can be applied to the cosmetic. The water in the cosmetic of the present invention is included as one of the components in a water-phase composition constituting a water phase.

The content of the water in the water-in-oil cosmetic of the present invention is preferably in a range of 1% by mass to 90% by mass, more preferably in a range of 10% by mass to 80% by mass, and still more preferably in a range of 30% by mass to 70% by mass of the total mass of the cosmetic.

The content of the water is preferably in a range of 30% by mass to 95% by mass, more preferably in a range of 40% by mass to 90% by mass, and still more preferably in a range of 45% by mass to 85% by mass of the total mass of the water-phase composition.

### (g) Other components

The water-in-oil cosmetic of the present invention is capable of including, in addition to the above-described components, a surfactant other than the sorbitan fatty acid ester, an oil-phase component such as a lipophilic thickener, and a water-phase component such as a water-soluble thickener as necessary.

### (Lipophilic thickener)

In a case in which the water-in-oil cosmetic of the present invention includes the surface-treated powder in an oil phase, the water-in-oil cosmetic preferably includes a lipophilic thickener. The inclusion of the lipophilic thickener is capable of further improving the stability of the oil phase in the -water-in-oil cosmetic. When the lipophilic thickener is made to coexist with the carotenoid in the same oil phase, it is possible to further enhance the temporal stability of the carotenoid. The lipophilic thickener refers to a component that has an action of dispersing in an oil phase so as to hold an ultraviolet ray-scattering agent and/or water and stably assisting the dispersion thereof and, furthermore, is capable of imparting viscosity to the oil phase.

The form of the lipophilic thickener may be any of a particle form, a paste form, a slurry form, a gum form, a liquid form, a crystal form, and the like, and, from the viewpoint of the temporal stability of the cosmetic, the form of the lipophilic thickener is preferably a particle form, a paste form, or a slurry form and more preferably a particle form. Here, the particle form includes a spherical shape, a plate shape, an irregular form, and the like which have the maximum diameter of 100 µm or smaller. The lipophilic thickener may be porous or have no pores therein.

In a case in which the lipophilic thickener has a particle form, the volume-average particle diameter of the lipophilic thickener is preferably in a range of 0.005 µm to 30 µm, more preferably in a range of 0.01 µm to 30 µm, and still more preferably in a range of 0.1 µm to 30 µm. When the volume-average particle diameter of the lipophilic thickener is 0.005 µm or larger, a user does not feel any roughness on the skin when using the water-in-oil cosmetic, and the feeling during use is further improved. When the volume-average particle diameter of the lipophilic thickener is 30 µm or smaller, there are no cases in which the surface area per unit weight of the lipophilic thickener becomes excessively low, and the adhesiveness to the skin tends not to be impaired. The volume-average particle diameter of the lipophilic thickener can be measured using a variety of commercially available particle size distribution meters or the like. For the measurement of the weight-average particle diameter of the lipophilic thickener, a dynamic light scattering method is preferably applied in consideration of the particle diameter range and ease of measurement. Examples of a commercially available determination device using dynamic light scattering include NANOTRAC UPA (manufactured by Nikkiso Co., Ltd.), a dynamic light scattering-type determination device of particle diameter distribution LB-550 (manufactured by Horiba Ltd.), and a denseness-based particle diameter analyzer FPAR-1000 (manufactured by Otsuka Electronics Co., Ltd.).

The volume-average particle diameter of the lipophilic thickener in the present invention is a value measured using a denseness-based particle diameter analyzer FPAR-1000 (manufactured by Otsuka Electronics Co., Ltd.), and specifically, a value measured as described below is employed.

That is, regarding the method for measuring the volume-average particle diameter of the lipophilic thickener, the lipophilic thickener is diluted with dimethicone so that the concentration thereof reaches 1% by mass, and the volume-average particle diameter is measured using a silica cell. The particle diameter of the lipophilic thickener can be obtained as the volume-average particle diameter when the refractive index of a specimen is set to 1.600, the refractive index of a dispersion medium is set to 1.000 (dimethicone), and the viscosity of the dispersion medium is set to the viscosity of dimethicone.

Examples of the lipophilic thickener include an organic modified clay mineral, silica powder, porous silica powder, crosslinking silicone powder, polymethyl methacrylate powder, corn starch, and a dextrin fatty acid ester. Only one lipophilic thickener may be used or a combination of two or more lipophilic thickeners may be used.

Examples of the organic modified clay mineral include dodecyl dimethyl benzyl ammonium montmorillonite and dimethyl dioctadecyl ammonium montmorillonite.

Examples of the silica powder include silica powder, silica silylate powder, silica dimethyl silylate powder, and silica dimethicone silylate powder.

Examples of the porous silica powder include porous silica powder, porous silica silylate powder, porous silica dimethyl silylate powder, and porous silica dimethicone silylate powder, and, from the viewpoint of stabilizing the viscosity, silica is preferred. Examples of commercially available products of the porous silica powder include SUNSPHERE H-31/H-32/H-33/H-51/H-52/H-53/H-121/H-122/H-201 (manufactured by AGC Si-Tech Co., Ltd.), VM-2270 Aerogel Fine Particle (manufactured by Dow Corning Toray Co., Ltd.), HDK (registered trademark) H2000, HDK (registered trademark) H15, HDK (registered trademark) H18, HDK (registered trademark) H20, and HDK (registered trademark) H30 (manufactured by Wacker Asahikasei Silicone Co., Ltd.), SYLOPURE (manufactured by Fuji Silysia Chemical Ltd.), TOKUSIL (manufactured by Tokuyama Corporation), and MICRO-BEADS SILICA GEL (manufactured by Fuji Davison Chemical Ltd.).

Examples of the crosslinking silicone powder include silicone gel powder, silicone rubber powder, silicone resin powder, and silicone elastomer, and these powders can be used without any distinction. From the viewpoint of the dispersibility of the cosmetic, the crosslinking silicone powder is preferably a dimethicone/vinyl dimethicone crosspolymer, a dimethicone/bis-isobutyl PPG-20 crosspolymer, a dimethicone crosspolymer, a dimethicone (PET-10) crosspolymer, a PEG-10/lauryl dimethicone crosspolymer, a PEG-10 dimethicone/vinyl dimethicone crosspolymer, a dimethicone (PEG-10/15) crosspolymer, a PEG-15/lauryl dimethicone crosspolymer, a PEG-15/lauryl polydimethylsiloxyethyl dimethicone crosspolymer, a polyglyceryl-3/lauryl polydimethylsiloxyethyl dimethicone crosspolymer, a dimethicone/polyglycerin-3 crosspolymer, a lauryl dimethicone/polyglycerin-3 crosspolymer, a polyglyceryl-3/lauryl polydimethylsiloxyethyl dimethicone crosspolymer, a diphenyl dimethicone/vinyl diphenyl dimethicone/silsesquioxane crosspolymer, a dimethicone/bis-isobutyl PPG-20 crosspolymer, a dimethicone/vinyltrimethylsiloxysilicate crosspolymer, a dimethicone/phenyl vinyl dimethicone crosspolymer, a vinyl dimethicone/lauryl dimethicone crosspolymer, a vinyl dimethicone/methicone silsesquioxane crosspolymer, a lauryl polydimethylsiloxyethyl dimethicone/bis-vinyldimethicone crosspolymer, or the like.

Examples of a commercially available product of the silicone gel powder include KSG-15/16/1610, KSG-18A, KSG-41, KSP-100/101/102/105, KSP-300, 441/411, KSG-41/42/43/44, and KSG-240/310/320/330/340/710/320Z/350Z (manufactured by Shin-Etsu Chemical Co., Ltd.). Examples of a commercially available product of the silicone rubber powder include TORAYFIL E-506C, E-508, 9701 Cosmetic Powder, 9702 Powder, 9027/9040/9041/9045/9046/9041/9546 Silicone Elastomer Blend, EP-9215/EP-9216 TI/EP-9289 LL/EP-9293 AL, EL-8040 ID Silicone Organic Blend (manufactured by Dow Corning Toray Co., Ltd.), Wacker-Belsil (registered trademark) RG 100 (manufactured by Wacker Asahikasei Silicone Co., Ltd.), and NIKKOL SILBLEND-91 (manufactured by Nikko Chemicals Co., Ltd.).

Examples of the polymethyl methacrylate (hereinafter, also simply referred to as "PMMA") powder include, in addition to PMMA, powders of a methyl methacrylate crosspolymer, a methyl methacrylate/glycol dimethacrylate crosspolymer, a methyl methacrylate/acrylonitrile copolymer, a polymethyl methacrylate/dimethyl polysiloxane graft acrylic resin copolymer, and an ethylhexyl acrylate/methyl methacrylate copolymer. Examples of a commercially available product of PMMA include TECHPOLYMER (manufactured by Sekisui Chemical Co., Ltd.) and MATSUMOTO MICROSPHERE (manufactured by Matsumoto Yushi-Seiyaku Co., Ltd.).

As the corn starch, DRY FLO PURE (manufactured by Akzo Nobel N.V) or the like can be used.

Examples of the dextrin fatty acid ester include dextrin palmitate, dextrin palmitate/2-ethyl hexanoate, dextrin myristate, dextrin stearate, dextrin plamitate/stearate, dextrin oleate, dextrin isopalmitate, and dextrin isostearate.

In addition, as the lipophilic thickener, a porous polymer made of styrene/stearyl methacrylate/divinyl benzene or the like, a hydrogenated (styrene/isoprene) copolymer, a butylene/ethylene/styrene copolymer, a styrene/acrylamide copolymer, an ethylene/propylene/styrene copolymer, a clay mineral, bentonite, synthetic phlogopite, a polyamide resin, flaky titanium oxide, or the like can also be used. Examples of a commercially available product of the lipophilic thickener include LUXELEN D (manufactured by Nihon Koken Kogyo Co., Ltd.), TIPAQUE CR-50 (manufactured by Ishihara Sangyo Kaisha, Ltd.), PIONEER GEL 12 PAO (manufactured by Hansen & Rosenthal KG), JOJOBA GLAZE HV/LV (manufactured by Nikko Chemicals Co., Ltd.), YODOSOL GH41F (manufactured by Akzo Nobel N.V), and KUNIPIA F/G (manufactured by Kunimine Industries Co., Ltd.).

From the viewpoint of the temporal stability of carotenoids, the temporal stability of the cosmetic, and improvement in the feeling of the cosmetic during use, the lipophilic thickener is preferably at least one lipophilic thickener selected from a group consisting of a dimethicone/vinyl dimethicone crosspolymer, and a vinyl dimethicone/methiconesilsesquioxane crosspolymer.

In a case in which the surface-treated powder is included in an oil phase, the lipophilic thickener may be included in the oil phase including the surface-treated powder or may be included in an oil phase that is different from the oil phase including the surface-treated powder. In a case in which the lipophilic thickener is included in the oil phase including the surface-treated powder, there is an advantage of improving the dispersibility of the surface-treated powder. In addition, in a case in which the lipophilic thickener is included in an oil phase that is different from the oil phase including the surface-treated powder, there is an advantage of stabilizing the oil phase and stabilizing the entire cosmetic.

The total content of the lipophilic thickener in the water-in-oil cosmetic is preferably set in a range of 0.005% by mass to 30% by mass, more preferably set in a range of 0.01% by mass to 20% by mass, and still more preferably set in a range of 0.05% by mass to 15% by mass of the total mass of the cosmetic in terms of improving the temporal stability of the cosmetic. When the total content of the lipophilic thickener is 0.005% by mass or higher of the total mass of the cosmetic, there is a tendency that the dispersibility of the oil phase improves, and when the total content thereof is 30% by mass or lower, the feeling of the cosmetic during use is favorable when applied, and there is a tendency that the spreading of the cosmetic improves.

### (Surfactant)

The cosmetic according to the present invention is capable of including a surfactant other than the above-described components such as the sorbitan fatty acid ester.

The surfactant other than the sorbitan fatty acid ester (hereinafter, simply referred to as "surfactant" unless particularly otherwise described) may be either an ionic surfactant such as an anionic surfactant, a cationic surfactant, or an amphoteric surfactant or a nonionic surfactant. Examples of the ionic surfactant include alkylsulfonate, alkyl sulfate, monoalkyl phosphoate, and lecithin. Examples of the nonionic surfactant include a glycerin fatty acid ester, an organic acid monoglyceride, a polyglycerin fatty acid ester, a propylene glycol fatty acid ester, a polyglycerin condensed ricinoleic acid ester, a sucrose fatty acid ester, a polyethylene glycol fatty acid ester, a polyoxyethylene alkyl ether, a polyoxypropylene alkyl ether, a polyoxyethylene alkyl phenyl ether, a polyoxyethylene fatty acid ester, a polyoxyethylene sorbitan fatty acid ester, a polyoxyethylene sorbitol fatty acid ester, a polyoxyethylene glycerin fatty acid ester, a polyoxyethylene propylene glycol fatty acid ester, a polyoxyethylene castor oil, and a polyoxyethylene hardened castor oil. Among these, a polyglycerin fatty acid ester, for example, an ester of polyglycerin having a degree of polymerization in a range of 2 to 15 and a linear or branched fatty acid having 6 to 22 carbon atoms is more preferred from the viewpoint of improving the dispersibility of the surface-treated powder.

The content of the surfactant is preferably set in a range of 0.01% by mass to 30% by mass, more preferably in a range of 0.05% by mass to 20% by mass, and still more preferably in a range of 0.1 % by mass to 10% by mass of the total mass of the cosmetic. When the content of the surfactant is 0.01% by mass or higher of the total mass of the cosmetic, there is a tendency that the temporal stability of the water-in-oil cosmetic becomes favorable, and, when the content thereof is 30% by mass or lower, there is a tendency that the feeling during use further improves.

### (Ultraviolet absorbents other than 4-tert-butyl-4-methoxy benzoyl methane)

The water-in-oil cosmetic is capable of including an ultraviolet absorbents other than the above-described 4-tert-butyl-4-methoxy benzoyl methane as long as the effect of the present invention is not impaired.

As the above-described ultraviolet absorbent, any of well-known oil-soluble or water-soluble ultraviolet absorbents can be used.

Examples of the oil-soluble ultraviolet absorbents include para-aminobenzoic acid, methyl para-aminobenzoate, glyceryl para-aminobenzoate, amyl para-dimethyl aminobenzoate, octyl para-dimethyl aminobenzoate, ethylene glycol salicylate, phenyl salicylate, octyl salicylate, butyl phenyl salicylate, homomentyl salicylate, octyl methoxycinnamate, ethoxyethyl methoxycinnamate, ethylhexyl methoxycinnamate, glyceryl monoethylhexanoate dimethoxycinnamate, hydroxymethoxybenzophenone, dihydroxy dimethoxybenzophenone, butyl methoxy benzoyl methane, and octyl triazone.

Examples of the water-soluble ultraviolet absorbent include benzophenone-based ultraviolet absorbents such as 2,4-dihydroxybenzophenone and 2,2'-dihydoxy-4-methoxybenzophenone; benzoimidazole-based ultraviolet absorbents such as phenylbenzimidazole-5-sulfonic acid, salts thereof, phenylene bisbenzoimidazole tetrasulfonic acid, and salts thereof; urocanic acid, urocanic acid ethyl ester, 2,2-(1,4-phenylene)bis-(1H-benzimidazole-4,6-disulfonic acid), and terephthalylidene dicamphor sulfonic acid.

In terms of compensating ultraviolet ray-defending performance, the content of the ultraviolet absorbent other than 4-tert-butyl-4-methoxy benzoyl methane is preferably set in a range of 0.001% by mass to 30% by mass, more preferably in a range of 0.01% by mass to 20% by mass, and still more preferably in a range of 0.1% by mass to 10% by mass of the total mass of the cosmetic. In a case in which the ultraviolet absorbent other than 4-tert-butyl-4-methoxy benzoyl methane is used while not impairing the effect of the present invention, the proportion of other ultraviolet absorbents in 4-tert-butyl-4-methoxy benzoyl methane can be set in a range of 0.01% by mass to 20% by mass and can be set in a range of 0.1% by mass to 10% by mass.

In terms of the feeling during use, the total amount of ultraviolet absorbents in the water-in-oil cosmetic is preferably set in a range of 0.001% by mass to 50% by mass, more preferably in a range of 0.01% by mass to 30% by mass, and still more preferably in a range of 0.1% by mass to 20% by mass of the total mass of the cosmetic.

### (Volatile oil component)

The water-in-oil cosmetic is capable of including a volatile oil component as a solvent. The inclusion of a volatile oil component as a solvent is capable of reducing a greasy feeling.

The volatile oil component refers to a component having a boiling point in a range of 60°C to 260°C at normal pressure. Examples of the volatile oil component used in the present invention include a volatile silicone-based oil and a hydrocarbon-based oil.

Examples of the volatile silicone-based oil include chain-like polysiloxanes such as dimethylpolysiloxane (1.5 cs [1.5×10⁻⁶ m²/s]), methyl phenyl polysiloxane, or methyl hydrogen polysiloxane, cyclic polysiloxane such as octamethyl cyclotetrasiloxane, cyclopentasiloxane, decamethylcyclopentasiloxane, dodecamethylcyclohexasiloxane, or tetramethyl tetrahydrogen cyclotetrasiloxane, and caprylyl methicone. Examples of a product thereof include KF96L-0.65, KF96L-1, KF96L1.5, KF995 (manufactured by Shin-Etsu Chemical Co., Ltd.), SH200-1cs, SH200-1.5cs, SH200-2cs, 2-1184Fluid, SH245Fluid, DC246Fluid, DC345Fluid, SS3408 (manufactured by Dow Corning Toray Co., Ltd.), TSF404, TSF405, and TSF4045 (manufactured by Momentive Performance Materials Inc.).

As the volatile hydrocarbon-based oil, a volatile hydrocarbon-based oil selected from a group consisting of linear and branched hydrocarbon-based oils may be used. Examples of the volatile hydrocarbon-based oil include C₈ to C₁₆ isoalkanes (also known as isoparaffins) such as isodecane, isododecane, and isohexadecane. Examples of a product thereof include ISOPAR (registered trademark) A, ISOPAR (registered trademark) C, ISOPAR (registered trademark) D, ISOPAR (registered trademark) E, ISOPAR (registered trademark) G, ISOPAR (registered trademark) H, ISOPAR (registered trademark) K, ISOPAR (registered trademark) L, and ISOPAR (registered trademark) M (manufactured by Exxon Mobil Corporation), SHELLSOL (registered trademark) 71 (manufactured by Royal Dutch Shell Plc), SALTROL (registered trademark) 100, SALTROL 130, and SALTROL (manufactured by Koninklijke Philips N.V), ISOZOL (registered trademark) 400 (manufactured by Nippon Petrochemicals Co., Ltd.), PARLEAM (registered trademark) 4 (manufactured by NOF Corporation), and IP SOLVENT (registered trademark) 1620, IP SOLVENT (registered trademark) 2028 (manufactured by Idemitsu Petrochemical Co., Ltd.), ISOHEXADECANE and TETRAISOBUTANE 90 (manufactured by Bayer AG), and PERMETHYL (registered trademark) 99A, PERMETHYL (registered trademark) 101A, and PERMETHYL (registered trademark) 102A (manufactured by Presperse LLC).

Only one volatile oil component may be used or a combination of two or more volatile oil components may be used.

In terms of the feeling during use, the content of the volatile oil component is preferably set in a range of 0.001% by mass to 60% by mass, more preferably in a range of 0.01 % by mass to 40% by mass, and still more preferably in a range of 0.1 % by mass to 20% by mass of the total mass of the cosmetic.

### (Polyhydric alcohol)

The water-in-oil cosmetic is capable of including a polyhydric alcohol. The inclusion of a polyhydric alcohol is capable of further improving the feeling during use (moisture-retaining properties).

Examples of the polyhydric alcohol include glycerin, 1,3-butanediol, ethylene glycol, polysaccharides, reduced starch syrup, sucrose, erythritol, xylitol, glucose, galactose, sorbitol, maltotriose, and trehalose. Only one polyhydric alcohol can be used or a combination of two or more polyhydric alcohols can be used.

Since moisture-retaining properties can be imparted, the content of the polyhydric alcohol is preferably set in a range of 0.01% by mass to 20% by mass, more preferably in a range of 0.05% by mass to 15% by mass, and still more preferably in a range of 0.1% by mass to 10% by mass of the total mass of the cosmetic.

### (Other components)

The water-in-oil cosmetic is capable of including other components that are ordinarily used in cosmetics on the basis of use. Examples of the above-described components include a water-soluble organic solvent such as ethanol, a chelating agent, a whitening agent, an emollient such as isotridecyl isononanoate, a moisturizer, an antioxidant, a hydrophilic thickener, a coloring agent, a preservative, a perfume, a variety of oily components, and a variety of aqueous components.

### (Manufacturing method)

The water-in-oil cosmetic can be manufactured using a manufacturing method in which an oil-phase composition including the above-described respective components and a water-phase composition are mixed together and the mixture is emulsified using an ordinary method.

When the surface-treated powder is formulated as the oil-phase component, the water-in-oil cosmetic can be manufactured by combining the surface-treated powder with other oil-phase components so as to prepare the oil-phase composition and combining the obtained oil-phase composition with the water-phase composition.

In a case in which the surface-treated powder is formulated as the water-phase component, the water-in-oil cosmetic can be manufactured by combining the surface-treated powder with other water-phase components so as to prepare the water-phase composition and combining the obtained water-phase composition with the oil-phase composition. Alternatively, the water-in-oil cosmetic can be manufactured by preparing a water-phase composition not including the surface-treated powder, combining the obtained water-phase composition with the oil-phase composition, preparing a preliminary water-in-oil composition not including the surface-treated powder, and adding the surface-treated powder to a water phase of the obtained preliminary water-in-oil composition. When the surface-treated powder is added to the water phase of the preliminary water-in-oil composition, the surface-treated powder may be singly added thereto, or it is also possible to combine the surface-treated powder with other water-phase components so as to prepare a water-phase composition and add the surface-treated powder as one component of the obtained water-phase composition.

Regarding an emulsification method for obtaining the water-in-oil cosmetic in the form of a water-in-oil emulsified substance by combining the oil-phase composition and the water-phase composition, there is no particular limitation, and an ordinary method can be used.

When the emulsified substance is prepared, the ratio (mass) of the oil-phase composition to the water-phase composition is not particularly limited, but the oil phase/water phase ratio (% by mass) is preferably in a range of 5/95 to 90/10, more preferably in a range of 10/90 to 80/20, and still more preferably in a range of 15/85 to 70/30.

### (Form)

The form of the water-in-oil cosmetic is not particularly limited, and examples thereof include a skincare cosmetic such as an emulsion, a cream, an eye cream, a serum, a massage material, a pack material, a spray, an ointment, or a body cream, a makeup cosmetic such as a makeup base, and a scalp cosmetic such as a leave-on essence.

The water-in-oil cosmetic of the present invention has a favorable ultraviolet ray shielding function and a favorable feeling during use and is thus particularly preferably used as a cosmetic for a sunblock.

### (Temporal stability of carotenoid)

The water-in-oil cosmetic of the present invention has high temporal stability of carotenoids. The temporal stability of carotenoids can be evaluated by irradiating a test specimen (10 g) with simulated sunlight with an intensity of 50 W/m² using a light resistance tester (Q-Sun Xe-3 manufactured by Q-LAB Corporation) at a distance of 20 cm for 120 minutes, and measuring the absorbance (480 nm) using a spectrophotometer.

Specifically, the absorbance of the test specimen before being irradiated with simulated sunlight, the absorbance of the test specimen irradiated with simulated sunlight for 5 minutes, and the absorbance of the test specimen irradiated with simulated sunlight for 120 minutes are respectively measured, and the percentage of the absorbance of the test specimen irradiated with simulated sunlight for 5 minutes or the absorbance of the test specimen irradiated with simulated sunlight for 120 minutes with respect to the absorbance of the test specimen before being irradiated with simulated sunlight is considered as the residual percentage of the carotenoid. When the residual percentage after 5 minutes of irradiation is 50% or higher and the residual percentage after 120 minutes of irradiation is 10% or higher, the temporal stability of carotenoids is evaluated to be high. Regarding the temporal stability of carotenoids in the water-in-oil cosmetic, it is preferable that the residual percentage after 5 minutes of irradiation is 60% or higher and the residual percentage after 120 minutes of irradiation is 30% or higher, and it is more preferable that the residual percentage after 5 minutes of irradiation is 70% or higher and the residual percentage after 120 minutes of irradiation is 50% or higher.

### (Temporal stability of cosmetic)

The water-in-oil cosmetic of the present invention preferably exhibits excellent temporal stability. The temporal stability of the water-in-oil cosmetic refers to the suppression of a change in the properties of the water-in-oil cosmetic for a certain period of time after the manufacture of the water-in-oil cosmetic. In the present invention, examples of the change in the properties include the aggregation of the surface-treated powder, and phase separation and/or gelation in the emulsified substance.

### (Feeling during use)

The water-in-oil cosmetic of the present invention exhibits a favorable feeling during use. In the present invention, the feeling during use refers to, for example, the favorable condition of the facial state after makeup. After the water-in-oil cosmetic is applied to the face, it is visually evaluated whether or not unevenness caused by uneven elements of the skin such as wrinkles or skin pores is barely visible. In a case in which uneven elements of the skin are covered and thus the skin appears to be even, the facial state after makeup is determined to be favorable.

### Examples

Hereinafter, the present invention will be described in detail using examples. However, the present invention is not limited thereto.

### [Examples 1 to 4]

An oil-phase composition and a water-phase composition were prepared using an a component, a b component, a c component, a d component, an e component, and other components so that the final concentrations (% by mass) shown in Table 1 below were obtained, and emulsification was carried out according to an ordinary method using the obtained oil-phase composition and water-phase composition, thereby obtaining a water-in-oil cosmetic. Individual components shown in Table 1 will be described below in detail.

Meanwhile, in each of Examples 1 and 2, the a component was mixed into cyclopentasiloxane using a disper, and other oil-phase components were combined with the mixture, thereby preparing an oil-phase composition. After that, the oil-phase composition and the water-phase composition were combined together at a ratio (mass ratio) of an oil phase to a water phase of 28.5:71.5, and emulsification was carried out using a homomixer under conditions of 25°C for 5 minutes at 6000 rpm, thereby obtaining a water-in-oil cosmetic. In the water-in-oil cosmetic of each of Examples 1 and 2, the a component is formulated into the oil phase.

In each of Examples 3 and 4, the a component was mixed into purified water and 1,3-butylene glycol (BG) using a disper, and other water-phase components were combined with the mixture, thereby preparing a water-phase composition. After that, the water-phase composition and the oil-phase composition were combined together at a ratio (mass ratio) of an oil phase to a water phase of 22.5:77.5, and emulsification was carried out using a homomixer under conditions of 25°C for 5 minutes at 6000 rpm, thereby obtaining a water-in-oil cosmetic. In the water-in-oil cosmetic of each of Examples 3 and 4, the a component is formulated into the water phase.

### [Comparative Examples 1 and 2]

Water-in-oil cosmetics were obtained in the same manner as in Example 1 except for the fact that, instead of the a component, silicone-coated titanium oxide and 4-tert-butyl-4-methoxy benzoyl methane (Comparative Example 1) and only silicone-coated titanium oxide (Comparative Example 2) were used, and silicone-coated titanium oxide and 4-tert-butyl-4-methoxy benzoyl methane were respectively formulated into the oil phase.

### [Comparative Examples 3 and 4]

Water-in-oil cosmetics were obtained in the same manner as in Example 3 except for the fact that, instead of the a component, silica-coated titanium oxide and 4-tert-butyl-4-methoxy benzoyl methane (Comparative Example 3) and only silica-coated titanium oxide (Comparative Example 4) were used, and silica-coated titanium oxide and 4-tert-butyl-4-methoxy benzoyl methane were respectively formulated into the water phase and the oil phase.

### [Examples 5 to 16 and Comparative Examples 5 to 10]

Water-in-oil emulsions were obtained in the same manner as in Example 1 except for the fact that the kinds and contents of individual components were changed as shown in Tables 2 and 3. Meanwhile, the individual components shown in Tables 2 and 3 will be described below in detail.

### [Examples 17 to 28 and Comparative Examples 11 to 16]

Water-in-oil emulsions were obtained in the same manner as in Example 3 except for the fact that the kinds and contents of individual components were changed as shown in Tables 4 and 5. Meanwhile, the individual components shown in Tables 4 and 5 will be described below in detail.

### [Examples 29 to 31]

Water-in-oil emulsions were obtained in the same manner as in Example 1 except for the fact that the kinds and contents of individual components were changed as shown in Table 6. Meanwhile, the individual components shown in Table 6 will be described below in detail.

### [Examples 32 to 34]

Water-in-oil cosmetics were obtained in the same manner as in Example 3 except for the fact that the kinds and contents of individual components were changed as shown in Table 6. Meanwhile, the individual components shown in Table 6 will be described below in detail.

### [Evaluations]

The following evaluations were carried out on individual test specimens of Examples 1 to 34 and Comparative Examples 1 to 16 obtained above. The results are shown in Tables 1 to 6. Meanwhile, regarding the comprehensive evaluations, except for the evaluations of the temporal stability of the emulsified substance, test specimens with no C or D grades were evaluated to be "Pass", that is, to have no practical problems.

### (1) Temporal stability of carotenoid

The test specimen (10 g) of each of Examples 1 to 34 and Comparative Examples 1 to 16 was put into a glass-based dish with a diameter (φ) of 35 mm and was irradiated with simulated sunlight with an intensity of 50 W/m² using a light resistance tester (Q-Sun Xe-3 manufactured by Q-LAB Corporation) in an environment of 25°C at a distance of 20 cm for 120 minutes. The absorbance (480 mm) was measured using an integrating sphere in a spectrophotometer before the irradiation, after 5 minutes of the irradiation, and after 120 minutes of the irradiation, and the percentage of the absorbance after 5 minutes of the irradiation or the absorbance after 120 minutes of the irradiation with respect to the absorbance before the irradiation was considered as the residual percentage of the carotenoid after 5 minutes of irradiation or 120 minutes of irradiation. The temporal stability of carotenoids was evaluated on the basis of the obtained residual percentages of carotenoids using the following indexes.
A: The residual percentage after 5 minutes of irradiation was 60% or higher, and the residual percentage after 120 minutes of irradiation was 30% or higher.
B: The residual percentage after 5 minutes of irradiation was in a range of 50% to lower than 60%, and the residual percentage after 120 minutes of irradiation was in a range of 10% to lower than 30%.
C: The residual percentage after 5 minutes of irradiation was in a range of 50% to lower than 60%, or the residual percentage after 120 minutes of irradiation was in a range of 10% to lower than 30%.
D: The residual percentage after 5 minutes of irradiation was lower than 50%, and/or the residual percentage after 120 minutes of irradiation was lower than 10%.

### (2) Dispersibility (initial stability of emulsified substance)

The test specimen (5 mg) of each of Examples 1 to 34 and Comparative Examples 1 to 16 was sandwiched between glass slides and was observed using an optical microscope (at a magnification of 350 times), and the presence or absence and emulsification state of an aggregate were visually evaluated using the following indexes.

Regarding the following indexes, in a case in which a plurality of 10 µm or larger black lumps could be observed in a single view, it is determined that "an aggregate is observed", in a case in which the sizes of the black lumps are in a range of 10 µm to 50 µm, it is determined that "a small aggregate is observed", and, in a case in which the sizes of the black lumps exceed 50 µm, it is determined that "a large aggregates is observed".

In addition, in a case in which, in a single view, emulsified particles with (a long diameter of) shorter than 1 µm are evenly dispersed, the test specimen is determined to be in a "homogeneous emulsification state", and, in a case in which there are emulsified particles with different sizes or emulsified particles are significantly unevenly dispersed, the test specimen is determined to be in a "non-homogeneous emulsification state".
A: A homogeneous emulsification state not including any aggregates.
B: A homogeneous emulsification state including a small number of aggregates.
C: An non-homogeneous emulsification state including a small number of aggregates.
D: A large aggregate is observed.

### (3) Temporal stability of emulsified substance (gelation)

The test specimen (50 g) of each of Examples 1 to 34 and Comparative Examples 1 to 16 was put into a vial, and an acceleration test was carried out in a constant temperature bath at 50°C for one month. In order to evaluate the stability of the emulsified substance, in the specimen subjected to the test, the occurrence of gelation and phase separation was observed after one week from the initiation of the test (hereinafter, simply referred to as "after one week") and after one month from the initiation of the test (hereinafter, simply referred to as "after one month") respectively, and the temporal stability of the emulsified substance was evaluated using the following indexes.

The occurrence of gelation was evaluated by skimming the specimen using a spatula and visually observing whether the specimen had been solidified.

Phase separation was evaluated by visually observing the top surface of the specimen from the outside of the bottle and visually observing whether or not liquid oozed out from the top surface of the specimen or whether or not the specimen integrally flowed without being separated when the vial was inclined.
A: Gelation and phase separation were not observed after one week and after one month.
B: Gelation was not observed after one week and after one month. Phase separation was not observed after one week, but was observed after one month.
C: Both gelation and phase separation were not observed after one week, but were observed after one month.
D: Both gelation and phase separation were observed after one week.

### (4) Evaluation of ultraviolet ray shielding

On the basis of the in vitro measurement method of ISO24443, the specimen (32.5 mg) of each of Examples 1 to 4 and Comparative Examples 1 to 4 was applied onto a PMMA plate (HELIOPLATE HD6) using a finger with an even thickness, the transmittance was measured using an SPF analyzer (UV-2000S manufactured by Labsphere, Inc.), and the UVB and UVA-defending effect was evaluated using the following standards.

### (4-1) (Standards for evaluating UVB-depending effect)

A: The SPF value is 20 or higher.
B: The SPF value is in a range of 15 to lower than 20.
C: The SPF value is in a range of 10 to lower than 15.
D: The SPF value is lower than 10.

### (4-2) (Standards for evaluating UVA-depending effect)

A: The transmittance at 400 nm is lower than 40%.
B: The transmittance at 400 nm is in a range of 40% to lower than 70%.
C: The transmittance at 400 nm is in a range of 70% to lower than 90%.
D: The transmittance at 400 nm is 90% or higher.

### (5) Facial state after makeup

20 persons in an expert panel for evaluating the cosmetic were asked to apply the test specimen (0.3 g) of each of Examples 1 to 34 and Comparative Examples 1 to 16 to half of the face, the appearance of the unevenness of the skin caused by wrinkles, fine wrinkles, and skin pores on the side of the face to which the cosmetic was applied was compared with that on the side of the face to which the cosmetic was not applied, and the facial state after makeup was evaluated using the following standards.
AA: Not visible
A: Barely visible
B: Slightly visible
C: Undeterminable
D: Clearly visible

As the respective components shown in Tables 1 to 6, the following substances were used. Meanwhile, numerical values for the respective components in Tables 1 to 6 have a unit of % by mass.

### (Surface-treated powder)

- 4-Tert-butyl-4-methoxy benzoyl methane-coated titanium oxide: HXMT-10EXA, manufactured by TAYCA Corporation (primary average particle diameter: 10 µm)
- Silicone-coated titanium oxide: MTY-02, manufactured by TAYCA Corporation (primary average particle diameter: 10 µm)
- Silica-coated titanium oxide: MT-05, manufactured by TAYCA Corporation (primary average particle diameter: 10 µm)
- Astaxanthin: ASTOTS-S, manufactured by Takedashiki Co., Ltd.
- Lycopene: Lyc-o-Mate, manufactured by Lycored
- PEG-9 polydimethylsiloxyethyl dimethicone: KF-6028P, manufactured by Shin-Etsu Chemical Co., Ltd., HLB: 4.0
- Lauryl PEG-9 polydimethylsiloxyethyl dimethicone: KF-6038, manufactured by Shin-Etsu Chemical Co., Ltd., HLB: 3.0
- PEG/PPG-19/19 dimethicone: BY11-030, manufactured by Dow Corning Toray Co., Ltd.
- PEG-10 dimethicone: KF-6017P, manufactured by Shin-Etsu Chemical Co., Ltd.
- Dimethicone/(PEG-10/15) crosspolymer: KSG-240, manufactured by Shin-Etsu Chemical Co., Ltd.
- Dimethicone/polyglyceryl-3 crosspolymer: KSG-710, manufactured by Shin-Etsu Chemical Co., Ltd.
- Trimethyl siloxysilicate: X-21-5249, manufactured by Shin-Etsu Chemical Co., Ltd.
- Sorbitan oleate: NIKKOL SO-10V, manufactured by Nikko Chemicals Co., Ltd.
- Sorbitan isostearate: NIKKOL SI-10RV, manufactured by Nikko Chemicals Co., Ltd.
- Sorbitan sesquioleate: NIKKOL SO-15V, manufactured by Nikko Chemicals Co., Ltd.
- Glyceryl stearate: NIKKOL MGS-AV, manufactured by Nikko Chemicals Co., Ltd.
- Polymethylsilsesquioxane: KMP-590, manufactured by Shin-Etsu Chemical Co., Ltd.

**[Table 1]**

| | No. | Components | Example 1 | Example 2 | Comparative Example 1 | Comparative Example 2 | Example 3 | Example 4 | Comparative Example 3 | Comparative Example 4 |
|---|---|---|---|---|---|---|---|---|---|---|
| a | 1 | Surface-treated powder (oil-phase formulation) | | | | | | | | |
| | | Titanium oxide | 5 | 2.5 | | | 5 | 2.5 | | |
| | | 4-Tert-butyl-4-methoxy benzoyl methane | 1 | 0.5 | | | 1 | 0.5 | | |
| a | 2 | Surface-treated powder (water-phase formulation) | | | | | | | | |
| | | Zinc oxide | | 2.5 | | | | 2.5 | | |
| | | 4-Tert-butyl-4-methoxy benzoyl methane | | 0.5 | | | | 0.5 | | |
| | 3 | Silicone-coated titanium oxide | | | 5 | 5 | | | | |
| | 4 | Silica-coated titanium oxide | | | | | | | 5 | 5 |
| | 5 | 4-Tert-butyl-4-methoxy benzoyl methane | | | 1 | | | | 1 | |
| b | 6 | Astaxanthin | 0.01 | 0.01 | 0.01 | 0.01 | 0.01 | 0.01 | 0.01 | 0.01 |
| c | 7 | Sorbitan oleate | 0.5 | 0.5 | 0.5 | 0.5 | 0.5 | 0.5 | 0.5 | 0.5 |
| d | 8 | PEG-9 polydimethylsiloxyethyl dimethicone | 3 | 3 | 3 | 3 | 3 | 3 | 3 | 3 |
| e | 9 | Trimethylsiloxysilicate | 1 | 1 | 1 | 1 | 1 | 1 | 1 | 1 |
| | 10 | BG | 3 | 3 | 3 | 3 | 3 | 3 | 3 | 3 |
| | 11 | Methyl paraben | 0.2 | 0.2 | 0.2 | 0.2 | 0.2 | 0.2 | 0.2 | 0.2 |
| | 12 | Sodium chloride | 0.5 | 0.5 | 0.5 | 0.5 | 0.5 | 0.5 | 0.5 | 0.5 |
| | 13 | Ethanol | 5 | 5 | 5 | 5 | 5 | 5 | 5 | 5 |
| | 14 | Isotridecyl isononanoate | 3 | 3 | 3 | 3 | 3 | 3 | 3 | 3 |
| | 15 | Cyclopentasiloxane | 15 | 15 | 15 | 16 | 15 | 15 | 15 | 16 |
| | 16 | Purified water | Residual amount | Residual amount | Residual amount | Residual amount | Residual amount | Residual amount | Residual amount | Residual amount |

| Evaluation items and determination results | | | | | | | | | | |
|---|---|---|---|---|---|---|---|---|---|---|
| (1)-1 | | Temporal stability of carotenoids, after 5 minutes of irradiation | 65% | 67% | 60% | 28% | 80% | 79% | 68% | 67% |
| (1)-2 | | Temporal stability of carotenoids, after 120 minutes of irradiation | 33% | 36% | 8% | 7% | 49% | 48% | 27% | 5% |
| (1) | | Temporal stability of carotenoids, comprehensive result | A | A | C | D | A | A | B | C |
| (2) | | Dispersibility | A | A | A | A | A | A | A | A |
| (3) | | Temporal stability of emulsion | A | A | A | A | A | A | A | A |
| (4-1) | | UV performance UVB | A | B | B | B | A | B | B | B |
| (4-2) | | UV performance UVA | A | A | C | D | A | A | C | D |
| (5) | | Facial state after makeup | A | A | B | A | A | A | B | A |

**[Table 2]**

| | No. | Components | Example 1 | Example 5 | Example 6 | Example 7 | Example 8 | Example 9 | Example 10 | Example 11 | Example 12 | Example 13 | Example 14 | Example 15 | Example 16 |
|---|---|---|---|---|---|---|---|---|---|---|---|---|---|---|---|
| a | 1 | Surface-treated powder (oil-phase formulation | | | | | | | | | | | | | |
| | | Titanium oxide | 5 | 5 | 5 | 5 | 5 | 5 | 5 | 5 | 5 | 5 | 5 | 5 | 5 |
| | | 4-Tert-butyl-4-methoxy benzoyl methane | 1 | 1 | 1 | 1 | 1 | 1 | 1 | 1 | 1 | 1 | 1 | 1 | 1 |
| b | 2 | Astaxanthin | 0.01 | 0.01 | 0.01 | 0.01 | 0.01 | 0.01 | 0.01 | 0.01 | 0.01 | 0.01 | 0.01 | 0.01 | 0.01 |
| c | 3 | Sorbitan oleate | 0.50 | | | 0.50 | 0.50 | 0.50 | 0.50 | 0.50 | 0.50 | 0.50 | 0.50 | 0.50 | 0.50 |
| | 4 | Sorbitan isostearate | | 0.50 | | | | | | | | | | | |
| | 5 | Sorbitan sesquioleate | | | 0.50 | | | | | | | | | | |
| | 6 | Glyceryl stearate | | | | | | | | | | | | | |
| d | 7 | PEG-9 polydimethylsiloxyethyl dimethicone | 3 | 3 | 3 | | | | 3 | 3 | 3 | 3 | 3 | 3 | 3 |
| | 8 | Lauryl PEG-9 polydimethylsiloxyethyl dimethicone | | | | 3 | | | | | | | | | |
| | 9 | PEG/PPG-19/19 dimethicone | | | | | 3 | | | | | | | | |
| | 10 | PEG-10 dimethicone | | | | | | 3 | | | | | | | |
| | 11 | Dimethicone/(PEG-10/15) crosspolymer | | | | | | | | | | | | | |
| | 12 | Dimethicone/polyglycerin-3 crosspolymer | | | | | | | | | | | | | |
| e | 13 | Trimethylsiloxysilicate | 2 | 2 | 2 | 2 | 2 | 2 | 0.1 | 12 | 20 | | | | |
| | 14 | Polymethylsilsesquioxane | | | | | | | | | | 2 | | | |
| | 15 | Alkyl acrylate/dimethicone copolymer | | | | | | | | | | | 2 | | |
| | 16 | Alkyl acrylate copolymer | | | | | | | | | | | | 2 | |
| | 17 | Dimethicone crosspolymer | | | | | | | | | | | | | 2 |
| | 18 | BG | 3 | 3 | 3 | 3 | 3 | 3 | 3 | 3 | 3 | 3 | 3 | 3 | 3 |
| | 19 | Methyl paraben | 0.2 | 0.2 | 0.2 | 0.2 | 0.2 | 0.2 | 0.2 | 0.2 | 0.2 | 0.2 | 0.2 | 0.2 | 0.2 |
| | 20 | Sodium chloride | 0.5 | 0.5 | 0.5 | 0.5 | 0.5 | 0.5 | 0.5 | 0.5 | 0.5 | 0.5 | 0.5 | 0.5 | 0.5 |
| | 21 | Ethanol | 5 | 5 | 5 | 5 | 5 | 5 | 5 | 5 | 5 | 5 | 5 | 5 | 5 |
| | 22 | Isotridecyl isononanoate | 3 | 3 | 3 | 3 | 3 | 3 | 3 | 3 | 3 | 3 | 3 | 3 | 3 |
| | 23 | Cyclopentasiloxane | 15 | 15 | 15 | 15 | 15 | 15 | 15 | 15 | 15 | 15 | 15 | 15 | 15 |
| | 24 | Purified water | Residual amount | Residual amount | Residual amount | Residual amount | Residual amount | Residual amount | Residual amount | Residual amount | Residual amount | Residual amount | Residual amount | Residual amount | Residual amount |

| Evaluation items and determination results | | | | | | | | | | | | | | | |
|---|---|---|---|---|---|---|---|---|---|---|---|---|---|---|---|
| (1) | | Temporal stability of carotenoids, comprehensive result | A | A | A | A | A | A | A | A | A | A | A | A | A |
| (2) | | Dispersibility | A | A | A | A | A | A | A | A | A | A | A | A | A |
| (3) | | Temporal stability of emulsion | A | A | A | A | B | B | A | A | A | A | A | A | A |
| (5) | | Facial state after makeup | A | A | A | A | A | A | A | A | B | A | A | A | B |

**[Table 3]**

| | No. | Components | Comparative Example 5 | Comparative Example 6 | Comparative Example 7 | Comparative Example 8 | Comparative Example 9 | Comparative Example 10 |
|---|---|---|---|---|---|---|---|---|
| a | 1 | Surface-treated powder (oil-phase formulation) | | | | | | |
| | | Titanium oxide | 5 | 5 | 5 | 5 | 5 | 5 |
| | | 4-Tert-butyl-4-methoxy benzoyl methane | 1 | 1 | 1 | 1 | 1 | 1 |
| b | 2 | Astaxanthin | 0.01 | 0.01 | 0.01 | 0.01 | 0.01 | 0.01 |
| C | 3 | Sorbitan oleate | | | 0.50 | 0.50 | 0.50 | 0.50 |
| | 4 | Sorbitan isostearate | | | | | | |
| | 5 | Sorbitan sesquioleate | | | | | | |
| | 6 | Glyceryl stearate | | 0.50 | | | | |
| d | 7 | PEG-9 polydimethylsiloxyethyl dimethicone | 3 | 3 | | | | 3 |
| | 8 | Lauryl PEG-9 polydimethylsiloxyethyl dimethicone | | | | | | |
| | 9 | PEG/PPG-19/19 dimethicone | | | | | | |
| | 10 | PEG-10 dimethicone | | | | | | |
| | 11 | Dimethicone/(PEG-10/15) crosspolymer | | | | 3 | | |
| | 12 | Dimethicone/polyglycerin-3 crosspolymer | | | | | 3 | |
| e | 13 | Trimethylsiloxysilicate | 2 | 2 | 2 | 2 | 2 | |
| | 14 | Polymethylsilsesquioxane | | | | | | |
| | 15 | Alkyl acrylate/dimethicone copolymer | | | | | | |
| | 16 | Alkyl acrylate copolymer | | | | | | |
| | 17 | Dimethicone crosspolymer | | | | | | |
| | 18 | BG | 3 | 3 | 3 | 3 | 3 | 3 |
| | 19 | Methyl paraben | 0.2 | 0.2 | 0.2 | 0.2 | 0.2 | 0.2 |
| | 20 | Sodium chloride | 0.5 | 0.5 | 0.5 | 0.5 | 0.5 | 0.5 |
| | 21 | Ethanol | 5 | 5 | 5 | 5 | 5 | 5 |
| | 22 | Isotridecyl isononanoate | 3 | 3 | 3 | 3 | 3 | 3 |
| | 23 | Cyclopentasiloxane | 15 | 15 | 15 | 15 | 15 | 15 |
| | 24 | Purified water | Residual | Residual | Residual | Residual | Residual | Residual |
| | | | amount | amount | amount | amount | amount | amount |
| Evaluation items and determination results | | | | | | | | |
| (1) | | Temporal stability of carotenoids, comprehensive result | B | A | B | B | B | A |
| (2) | | Dispersibility | D | B | A | A | A | B |
| (3) | | Temporal stability of emulsion | C | B | D | C | C | B |
| (5) | | Facial state after makeup | B | C | D | B | B | D |

**[Table 4]**

| | No. | Components | Example 3 | Example 17 | Example 18 | Example 19 | Example 20 | Example 21 | Example 22 | Example 23 | Example 24 | Example 25 | Example 26 | Example 27 | Example 28 |
|---|---|---|---|---|---|---|---|---|---|---|---|---|---|---|---|
| a | 1 | Surface-treated powder (water-phase formulation) | | | | | | | | | | | | | |
| | | Titanium oxide | 5 | 5 | 5 | 5 | 5 | 5 | 5 | 5 | 5 | 5 | 5 | 5 | 5 |
| | | 4-Tert-butyl-4-methoxy benzoyl methane | 1 | 1 | 1 | 1 | 1 | 1 | 1 | 1 | 1 | 1 | 1 | 1 | 1 |
| b | 2 | Astaxanthin | 0.01 | 0.01 | 0.01 | 0.01 | 0.01 | 0.01 | 0.01 | 0.01 | 0.01 | 0.01 | 0.01 | 0.01 | 0.01 |
| C | 3 | Sorbitan oleate | 0.50 | | | 0.50 | 0.50 | 0.50 | 0.50 | 0.50 | 0.50 | 0.50 | 0.50 | 0.50 | 0.50 |
| | 4 | Sorbitan isostearate | | 0.50 | | | | | | | | | | | |
| | 5 | Sorbitan sesquioleate | | | 0.50 | | | | | | | | | | |
| | 6 | Glyceryl stearate | | | | | | | | | | | | | |
| d | 7 | PEG-9 polydimethylsiloxyethyl dimethicone | 3 | 3 | 3 | | | | 3 | 3 | 3 | 3 | 3 | 3 | 3 |
| | 8 | Lauryl PEG-9 polydimethylsiloxyethyl dimethicone | | | | 3 | | | | | | | | | |
| | 9 | PEG/PPG-19/19 dimethicone | | | | | 3 | | | | | | | | |
| | 10 | PEG-10 dimethicone | | | | | | 3 | | | | | | | |
| | 11 | Dimethicone/(PEG-10/15) crosspolymer | | | | | | | | | | | | | |
| | 12 | Dimethicone/polyglycerin-3 crosspolymer | | | | | | | | | | | | | |
| e | 13 | Trimethylsiloxysilicate | 2 | 2 | 2 | 2 | 2 | 2 | 0.1 | 12 | 20 | | | | |
| | 14 | Polymethylsilsesquioxane | | | | | | | | | | 2 | | | |
| | 15 | Alkyl acrylate/dimethicone copolymer | | | | | | | | | | | 2 | | |
| | 16 | Alkyl acrylate copolymer | | | | | | | | | | | | 2 | |
| | 17 | Dimethicone crosspolymer | | | | | | | | | | | | | 2 |
| | 18 | BG | 3 | 3 | 3 | 3 | 3 | 3 | 3 | 3 | 3 | 3 | 3 | 3 | 3 |
| | 19 | Methyl paraben | 0.2 | 0.2 | 0.2 | 0.2 | 0.2 | 0.2 | 0.2 | 0.2 | 0.2 | 0.2 | 0.2 | 0.2 | 0.2 |
| | 20 | Sodium chloride | 0.5 | 0.5 | 0.5 | 0.5 | 0.5 | 0.5 | 0.5 | 0.5 | 0.5 | 0.5 | 0.5 | 0.5 | 0.5 |
| | 21 | Ethanol | 5 | 5 | 5 | 5 | 5 | 5 | 5 | 5 | 5 | 5 | 5 | 5 | 5 |
| | 22 | Isotridecyl isononanoate | 3 | 3 | 3 | 3 | 3 | 3 | 3 | 3 | 3 | 3 | 3 | 3 | 3 |
| | 23 | Cyclopentasiloxane | 15 | 15 | 15 | 15 | 15 | 15 | 15 | 15 | 15 | 15 | 15 | 15 | 15 |
| | 24 | Purified water | Residual amount | Residual amount | Residual amount | Residual amount | Residual amount | Residual amount | Residual amount | Residual amount | Residual amount | Residual amount | Residual amount | Residual amount | Residual amount |
| Evaluation items and determination results | | | | | | | | | | | | | | | |
| (1) | | Temporal stability of carotenoids, comprehensive result | A | A | A | A | A | A | A | A | A | A | A | A | A |
| (2) | | Dispersibility | A | A | A | A | A | A | A | A | A | A | A | A | A |
| (3) | | Temporal stability of emulsion | A | A | A | A | B | B | A | A | A | A | A | A | A |
| (5) | | Facial state after makeup | A | A | A | A | A | A | A | A | B | A | A | A | B |

**[Table 5]**

| | No. | Components | Comparative Example 11 | Comparative Example 12 | Comparative Example 13 | Comparative Example 14 | Comparative Example 15 | Comparative Example 16 |
|---|---|---|---|---|---|---|---|---|
| a | 1 | Surface-treated powder (water-phase formulation) | | | | | | |
| | | Titanium oxide | 5 | 5 | 5 | 5 | 5 | 5 |
| | | 4-Tert-butyl-4-methoxy benzoyl methane | 1 | 1 | 1 | 1 | 1 | 1 |
| b | 2 | Astaxanthin | 0.01 | 0.01 | 0.01 | 0.01 | 0.01 | 0.01 |
| C | 3 | Sorbitan oleate | | | 0.50 | 0.50 | 0.50 | 0.50 |
| | 4 | Sorbitan isostearate | | | | | | |
| | 5 | Sorbitan sesquioleate | | | | | | |
| | 6 | Glyceryl stearate | | 0.50 | | | | |
| d | 7 | PEG-9 polydimethylsiloxyethyl dimethicone | 3 | 3 | | | | 3 |
| | 8 | Lauryl PEG-9 polydimethylsiloxyethyl dimethicone | | | | | | |
| | 9 | PEG/PPG-19/19 dimethicone | | | | | | |
| | 10 | PEG-10 dimethicone | | | | | | |
| | 11 | Dimethicone/(PEG-10/15) crosspolymer | | | | 3 | | |
| | 12 | Dimethicone/polyglycerin-3 crosspolymer | | | | | 3 | |
| e | 13 | Trimethylsiloxysilicate | 2 | 2 | 2 | 2 | 2 | |
| | 14 | Polymethylsilsesquioxane | | | | | | |
| | 15 | Alkyl acrylate/dimethicone copolymer | | | | | | |
| | 16 | Alkyl acrylate copolymer | | | | | | |
| | 17 | Dimethicone crosspolymer | | | | | | |
| | 18 | BG | 3 | 3 | 3 | 3 | 3 | 3 |
| | 19 | Methyl paraben | 0.2 | 0.2 | 0.2 | 0.2 | 0.2 | 0.2 |
| | 20 | Sodium chloride | 0.5 | 0.5 | 0.5 | 0.5 | 0.5 | 0.5 |
| | 21 | Ethanol | 5 | 5 | 5 | 5 | 5 | 5 |
| | 22 | Isotridecyl isononanoate | 3 | 3 | 3 | 3 | 3 | 3 |
| | 23 | Cyclopentasiloxane | 15 | 15 | 15 | 15 | 15 | 15 |
| | 24 | Purified water | Residual amount | Residual amount | Residual amount | Residual amount | Residual amount | Residual amount |
| Evaluation items and determination results | | | | | | | | |
| (1) | | Temporal stability of carotenoids, comprehensive result | B | A | B | B | B | A |
| (2) | | Dispersibility | D | B | A | A | A | C |
| (3) | | Temporal stability of emulsion | C | B | D | C | C | B |
| (5) | | Facial state after makeup | B | D | C | B | C | D |

**[Table 6]**

| | No. | Components | Example 29 | Example 30 | Example 31 | Example 32 | Example 33 | Example 34 |
|---|---|---|---|---|---|---|---|---|
| a | 1 | Surface-treated powder (oil-phase formulation) | | | | | | |
| | | Titanium oxide | 12.5 | 16.7 | 12.5 | | | |
| | | 4-Tert-butyl-4-methoxy benzoyl methane | 2.5 | 3.3 | 2.5 | | | |
| a | 2 | Surface-treated powder (water-phase formulation) | | | | | | |
| | | Titanium oxide | | | | 12.5 | 16.7 | 12.5 |
| | | 4-Tert-butyl-4-methoxy benzoyl methane | | | | 2.5 | 3.3 | 2.5 |
| b | 3 | Astaxanthin | 0.01 | 0.01 | | 0.01 | 0.01 | |
| | 4 | Lycopene | | | 0.01 | | | 0.01 |
| c | 5 | Sorbitan oleate | 0.50 | 0.50 | 0.50 | 0.50 | 0.50 | 0.50 |
| d | 6 | PEG-9 polydimethylsiloxyethyl dimethicone | 3 | 3 | 3 | 3 | 3 | 3 |
| e | 7 | Trimethylsiloxysilicate | 2 | 2 | 2 | 2 | 2 | 2 |
| | 8 | BG | 3 | 3 | 3 | 3 | 3 | 3 |
| | 9 | Methyl paraben | 0.2 | 0.2 | 0.2 | 0.2 | 0.2 | 0.2 |
| | 10 | Sodium chloride | 0.5 | 0.5 | 0.5 | 0.5 | 0.5 | 0.5 |
| | 11 | Ethanol | 5 | 5 | 5 | 5 | 5 | 5 |
| | 12 | Isotridecyl isononanoate | 3 | 3 | 3 | 3 | 3 | 3 |
| | 13 | Cyclopentasiloxane | 15 | 15 | 15 | 15 | 15 | 15 |
| | 14 | Purified water | Residual amount | Residual amount | Residual amount | Residual amount | Residual amount | Residual amount |
| Evaluation items and determination results | | | | | | | | |
| (1) | | Temporal stability of carotenoids, comprehensive result | A | A | A | A | A | A |
| (2) | | Dispersibility | A | A | A | A | B | A |
| (3) | | Temporal stability of emulsion | A | A | A | A | C | A |
| (5) | | Facial state after makeup | A | A | A | A | A | A |

As shown in Tables 1 to 6, the water-in-oil cosmetics obtained by combining 4-tert-butyl-4-methoxy benzoyl methane-coated titanium oxide or 4-tert-butyl-4-methoxy benzoyl methane-coated zinc oxide, astaxanthin or lycopene as the carotenoid, a sorbitan fatty acid ester, PEG-9 polydimethylsiloxyethyl dimethicone or lauryl PEG-9 polydimethylsiloxyethyl dimethicone, and trimethyl siloxysilicate or nylon 12 all had favorable ultraviolet ray shielding functions and favorable temporal stability of carotenoids and the feeling during use and could be evaluated to be "Pass" in the comprehensive evaluation.

It was found that the above-described effects obtained from the examples could not be obtained in a case in which 4-tert-butyl-4-methoxy benzoyl methane was singly used (Comparative Examples 1 and 3), a case in which silicone-coated titanium oxide or silica-coated titanium oxide was used (Comparative Examples 2 and 4), and a case in which at least any of the sorbitan fatty acid ester, the polyether-modified silicone, the coating-forming silicone polymer, and the alkyl (meth)acrylate polymer was not included (Comparative Examples 5 to 16).

### [Example 35] Sunblock lotion spray (filled with nitrogen)

A sunblock lotion spray (filled with nitrogen) was prepared using an ordinary method according to the following formulation. The following numerical values indicate % by mass of the total mass of the formulation. Meanwhile, 4-tert-butyl-4-methoxy benzoyl methane-coated titanium oxide was formulated in the same manner as in Example 1 and was thus included in an oil phase.

| <Composition> | (% by mass) |
|---|---|
| • Propanediol | 0.6 |
| • Sorbitan oleate | 0.5 |
| • Methyl paraben | 0.6 |
| • Ethanol | 1.5 |
| • Phenyl methicone | 1.5 |
| • Isotridecyl isononanoate | 1.5 |
| • PEG-9 polydimethylsiloxyethyl dimethicone | 0.6 |
| • 4-Tert-butyl-4-methoxy benzoyl methane-coated titanium oxide | 15.0 |
| • Astaxanthin | 0.001 |
| • Cyclopentasiloxane | 15.0 |
| • Methicone | 0.9 |
| • Polymethylsilsesquioxane | 15.0 |
| • Trimethylsiloxysilicate | 0.6 |
| • Dimethicone | 1.5 |
| • Perfume | appropriate amount |
| • LPG | 75.0 |
| • Purified water | residual amount |

### [Example 36] Sunblock lotion spray (filled with nitrogen)

A sunblock lotion spray (filled with nitrogen) was prepared using an ordinary method according to the following formulation. The following numerical values indicate % by mass of the total mass of the formulation. Meanwhile, 4-tert-butyl-4-methoxy benzoyl methane-coated titanium oxide was formulated in the same manner as in Example 1 and was thus included in an oil phase.

| <Composition> | (% by mass) |
|---|---|
| • Propanediol | 0.6 |
| • Sorbitan oleate | 0.5 |
| • Methyl paraben | 0.6 |
| • Ethanol | 1.5 |
| • Phenyl methicone | 1.5 |
| • Isotridecyl isononanoate | 1.5 |
| • PEG-9 polydimethylsiloxyethyl dimethicone | 0.6 |
| • 4-Tert-butyl-4-methoxy benzoyl methane-coated titanium oxide | 15.0 |
| • Lycopene | 0.001 |
| • Cyclopentasiloxane | 15.0 |
| • Methicone | 0.9 |
| • Polymethylsilsesquioxane | 15.0 |
| • Trimethylsiloxysilicate | 0.6 |
| • Dimethicone | 1.5 |
| • Perfume | appropriate amount |
| • LPG | 75.0 |
| • Purified water | residual amount |

As described above, according to the present invention, it is possible to provide a water-in-oil cosmetic having a favorable ultraviolet ray shielding effect, an excellent temporal stability of carotenoids, and a favorable feeling during use.

The disclosure of JP2013-103487 is incorporated in its entirety into the present specification for reference.

All the publications, patent applications, and technical standards described in the present specification are incorporated into the present specification for reference to the same extent as the respective publications, patent applications, and technical standards and are specifically and individually described to be incorporated for reference.

## Claims

1. A water-in-oil cosmetic comprising:
surface-treated powder including at least one oxide selected from the group consisting of titanium oxide and zinc oxide and having 4-tert-butyl-4-methoxy benzoyl methane on a surface;
at least one carotenoid;
at least one sorbitan fatty acid ester;
at least one polyether-modified silicone; and
at least one compound selected from the group consisting of a coating-forming silicone polymer and an alkyl (meth)acrylate polymer.

2. The water-in-oil cosmetic according to claim 1, wherein the at least one polyether-modified silicone is a polyether-modified silicone having a straight chain or a branched chain.

3. The water-in-oil cosmetic according to claim 1 or 2, wherein the at least one polyether-modified silicone is a polyether-modified silicone having a branched chain.

4. The water-in-oil cosmetic according to any one of claims 1 to 3, wherein the at least one sorbitan fatty acid ester is at least one sorbitan fatty acid ester selected from the group consisting of sorbitan oleate, sorbitan isostearate, sorbitan sesquioleate, and sorbitan sesquiisostearate.

5. The water-in-oil cosmetic according to any one of claims 1 to 4, wherein the at least one polyether-modified silicone is at least one polyether-modified silicone selected from the group consisting of PET-9 polydimethylsiloxyethyl dimethicone and lauryl PET-9 polydimethylsiloxyethyl dimethicone.

6. The water-in-oil cosmetic according to any one of claims 1 to 5, wherein the at least one compound selected from the group consisting of a coating-forming silicone polymer and an alkyl (meth)acrylate polymer is at least one compound selected from the group consisting of trimethylsiloxysilicate, polymethylsilsesquioxane, an alkyl acrylate/dimethicone copolymer, and an alkyl acrylate copolymer.

7. The water-in-oil cosmetic according to any one of claims 1 to 6, wherein a total content of the at least one compound selected from the group consisting of a coating-forming silicone polymer and an alkyl (meth)acrylate polymer is in a range of 0.1% by mass to 15% by mass of the total mass of the water-in-oil cosmetic.

8. The water-in-oil cosmetic according to any one of claims 1 to 7, wherein the at least one carotenoid is at least one carotenoid selected from the group consisting of lycopene, astaxanthin, and derivatives thereof.

9. The water-in-oil cosmetic according to any one of claims 1 to 8, wherein the at least one carotenoid is astaxanthin.

10. The water-in-oil cosmetic according to claim 1, wherein a content of the surface-treated powder in the water-in-oil cosmetic is from 0.1% by mass to 30% by mass.

11. The water-in-oil cosmetic according to claim 1, wherein a total content of the at least one polyether-modified silicone in the water-in-oil cosmetic is from 0.01% by mass to 10% by mass of the total mass of the water-in-oil cosmetic.

## Patentansprüche

1. Wasser-in-Öl-Kosmetikum aufweisend:
Oberflächenbehandeltes Pulver, umfassend mindestens ein Oxid, das ausgewählt ist aus der Gruppe, die aus Titanoxid und Zinkoxid besteht, und das 4-tert-Butyl-4-methoxybenzoyl-methan an einer Oberfläche hat;
mindestens ein Carotinoid;
mindestens einen Sorbitanfettsäureester;
mindestens ein Polyether-modifiziertes Silikon; und
mindestens eine Verbindung, die ausgewählt ist aus der Gruppe, die aus einem beschichtungsbildenden Silikonpolymer und einem Alkyl(meth)acrylat-Polymer besteht.

2. Wasser-in-Öl-Kosmetikum nach Anspruch 1, wobei das mindestens eine Polyether-modifizierte Silikon ein Polyether-modifiziertes Silikon mit einer geraden Kette oder einer verzweigten Kette ist.

3. Wasser-in-Öl-Kosmetikum nach Anspruch 1 oder 2, wobei das mindestens eine Polyether-modifizierte Silikon ein Polyether-modifiziertes Silikon mit einer verzweigten Kette ist.

4. Wasser-in-Öl-Kosmetikum nach einem der Ansprüche 1 bis 3, wobei der mindestens eine Sorbitanfettsäureester mindestens ein Sorbitanfettsäureester ist, der ausgewählt ist aus der Gruppe, die aus Sorbitanoleat, Sorbitanisostearat, Sorbitansesquioleat und Sorbitansesquiisostearat besteht.

5. Wasser-in-Öl-Kosmetikum nach einem der Ansprüche 1 bis 4, wobei das mindestens eine Polyether-modifizierte Silikon mindestens ein Polyether-modifiziertes Silikon ist, das ausgewählt ist aus der Gruppe, die aus PET-9 Polydimethylsiloxyethyl-Dimethicone und Lauryl-PET-9 Polydimethylsiloxyethyl-Dimethicone besteht.

6. Wasser-in-Öl-Kosmetikum nach einem der Ansprüche 1 bis 5, wobei die mindestens eine Verbindung, die ausgewählt ist aus der Gruppe, die aus einem beschichtungsbildenden Silikonpolymer und einem Alkyl(meth)acrylat-Polymer besteht, mindestens eine Verbindung ist, die ausgewählt ist aus der Gruppe, die aus Trimethylsiloxysilikat, Polymethylsilsesquioxan, einem Alkyl-acrylat/Dimethicon-Copolymer und einem Alkyl-acrylat-Copolymer besteht.

7. Wasser-in-Öl-Kosmetikum nach einem der Ansprüche 1 bis 6, wobei ein Gesamtgehalt der mindestens einen Verbindung, die ausgewählt ist aus der Gruppe, die aus einem beschichtungsbildenden Silikonpolymer und einem Alkyl(meth)acrylat-Polymer besteht, in einem Bereich von 0,1 Masse-% bis 15 Masse-% der Gesamtmasse des Wasser-in-Öl-Kosmetikums liegt.

8. Wasser-in-Öl-Kosmetikum nach einem der Ansprüche 1 bis 7, wobei das mindestens eine Carotinoid mindestens ein Carotinoid ist, das ausgewählt ist aus der Gruppe, die aus Lycopen, Astaxanthin und Derivaten davon besteht.

9. Wasser-in-Öl-Kosmetikum nach einem der Ansprüche 1 bis 8, wobei das mindestens eine Carotinoid Astaxanthin ist.

10. Wasser-in-Öl-Kosmetikum nach Anspruch 1, wobei ein Gehalt des oberflächenbehandelten Pulvers in dem Wasser-in-Öl-Kosmetikum von 0,1 Masse% bis 30 Masse-% beträgt.

11. Wasser-in-Öl-Kosmetikum nach Anspruch 1, wobei ein Gesamtgehalt des mindestens einen Polyether-modifizierten Silikons in dem Wasser-in-Öl-Kosmetikum von 0,01 Masse-% bis 10 Masse-% der Gesamtmasse des Wasser-in-Öl-Kosmetikums beträgt.

## Revendications

1. Produit cosmétique huile dans eau, comprenant :
une poudre ayant subi un traitement de surface, incluant au moins un oxyde sélectionné parmi le groupe consistant en de l'oxyde de titane et de l'oxyde de zinc et présentant du 4-tert-butyl-4-méthoxybenzoyl-méthane sur une surface ;
au moins un caroténoïde ;
au moins un ester d'acide gras de sorbitan ;
au moins une silicone modifiée par un polyéther, et
au moins un composé sélectionné parmi le groupe consistant en un polymère de silicone formant un revêtement et un polymère de (méth)acrylate d'alkyle.

2. Produit cosmétique huile dans eau selon la revendication 1, dans lequel au moins une silicone modifiée par un polyéther est une silicone modifiée par un polyéther présentant une chaîne linéaire ou une chaîne ramifiée.

3. Produit cosmétique huile dans eau selon la revendication 1 ou 2, dans lequel la au moins une silicone modifiée par un polyéther est une silicone modifiée par un polyéther présentant une chaîne ramifiée.

4. Produit cosmétique huile dans eau selon l'une quelconque des revendications 1 à 3, dans lequel le au moins un ester d'acide gras de sorbitan est au moins un ester d'acide gras de sorbitan sélectionné parmi le groupe consistant en l'oléate de sorbitan, l'isostéarate de sorbitan, le sesquioléate de sorbitan, et le sesquiisostéarate de sorbitan.

5. Produit cosmétique huile dans eau selon l'une quelconque des revendications 1 à 4, dans lequel la au moins une silicone modifiée par un polyéther est au moins une silicone modifiée par un polyéther sélectionnée parmi le groupe consistant en PET-9 polydiméthylsiloxyéthyldiméthicone, et lauryl PET-9 polydiméthylsiloxyéthyldiméthicone.

6. Produit cosmétique huile dans eau selon l'une quelconque des revendications 1 à 5, dans lequel le au moins un composé sélectionné parmi le groupe consistant en un polymère de silicone formant un revêtement et un polymère de (méth)acrylate d'alkyle est au moins un composé sélectionné parmi le groupe consistant en du triméthylsiloxysilicate, du polyméthylsilsesquioxane, un copolymère d'acrylate d'alkyle/diméthicone et un copolymère d'acrylate d'alkyle.

7. Produit cosmétique huile dans eau selon l'une quelconque des revendications 1 à 6, dans lequel une teneur totale d'au moins un composé sélectionné parmi le groupe consistant en un polymère de silicone formant un revêtement et un polymère de (méth)acrylate d'alkyle est comprise dans une plage allant de 0,1 % en masse à 15 % en masse de la masse totale du produit cosmétique huile dans eau.

8. Produit cosmétique huile dans eau selon l'une quelconque des revendications 1 à 7, dans lequel le au moins un caroténoïde est au moins un caroténoïde sélectionné parmi le groupe consistant en du lycopène, l'astaxanthine, et des dérivés de ceux-ci.

9. Produit cosmétique huile dans eau selon l'une quelconque des revendications 1 à 8, dans lequel le au moins un caroténoïde est l'astaxanthine.

10. Produit cosmétique huile dans eau selon la revendication 1, dans lequel une teneur de la poudre ayant subi un traitement de surface dans le produit cosmétique huile dans eau est comprise dans une plage allant de 0,1 % en masse à 30 % en masse.

11. Produit cosmétique huile dans eau selon la revendication 1, dans lequel une teneur totale d'au moins une silicone modifiée par un polyéther dans le produit cosmétique huile dans eau est comprise dans une plage allant de 0,01 % en masse à 10 % en masse de la masse totale du produit cosmétique huile dans eau.
